(19) Europäisches Patentamt
European Patent Office
Office européen des brevets

(11) Publication number: 0 065 026
A1

# (12) EUROPEAN PATENT APPLICATION

(21) Application number: 81103810.8

(22) Date of filing: 18.05.81

(51) Int. Cl.³: C 07 C 125/06, B 01 J 27/08, B 01 J 31/02, B 01 J 31/04, B 01 J 31/26

(43) Date of publication of application: 24.11.82 Bulletin 82/47

(84) Designated Contracting States: BE DE FR GB NL

(71) Applicant: THE DOW CHEMICAL COMPANY, Dow Center 2030 Abbott Road Post Office Box 1967, Midland Michigan 48640 (US)

(72) Inventor: Gurgiolo, Arthur Emilio, 309 Oak Drive, Lake Jackson Texas 77466 (US)

(74) Representative: Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)

## (54) Preparation of carbamates from aromatic amines and organic carbonates.

(57) This invention is directed to a process for preparing a carbamate from an organic carbonate and an aromatic amine in the presence of catalytic quantities of a Lewis acid catalyst. The Lewis acid catalyst is soluble in the reaction mixture at the reaction conditions employed and is at least one member of the group consisting of a zinc or divalent tin halide, a zinc or divalent tin salt of a monovalent organic compound which has a pKa value of at least 2.8, and a zinc or divalent tin salt of trifluoroacetic acid.

EP 0 065 026 A1

0065026

# PREPARATION OF CARBAMATES FROM AROMATIC AMINES AND ORGANIC CARBONATES

Lewis acids have been disclosed in U.S. 3,763,217 as being suitable catalysts for reacting an organic carbonate with an aromatic amine to prepare carbamates.

It has been unexpectedly discovered that certain zinc and divalent tin salts provide the desired carbamates in higher yields and/or selectivity than the particular Lewis acids disclosed by Brill in U.S. 3,763, 217.

The present invention pertains to a process for preparing a carbamate from an organic carbonate and an aromatic amine in the presence of a catalytic quantity of a Lewis acid characterized by employing as the Lewis acid catalyst, a catalyst which is soluble in the reaction mixture at the reaction conditions employed and which is at least one member selected from the group consisting of a zinc or divalent tin halide, a zinc or divalent tin salt of a monovalent organic compound having a pKa value of at least 2.8, preferably from 4 to 10, and a zinc or divalent tin salt of trifluoroacetic acid.

Suitable organic carbonates which can be employed in the process of the present invention include the alkyl, aryl or alkyl aryl esters of carbonic acid. The ester group can be an alkyl group having up to 12 carbon atoms, preferably a lower alkyl group containing up to 6 carbon atoms or the ester group can be an aryl group containing up to 10 carbon atoms.

Particularly suitable organic carbonates are the cyclic and acyclic organic carbonates such as, for example, ethylene carbonate, propylene carbonate, styrene carbonate, dimethyl carbonate, diethyl carbonate, dipropyl carbonate, dibutyl carbonate, dihexyl carbonate, methyl ethyl carbonate, methyl butyl carbonate, diphenyl carbonate, methyl phenyl carbonate, and mixtures thereof.

Any aromatic amine having a conjugate pKa value of from 3.5 to 5.5 is suitable for use herein with those amines having a conjugate pKa value of from 4 to 5.4 being preferred.

Suitable such aromatic amines include those represented by the formulas

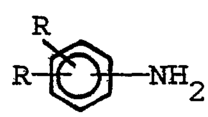

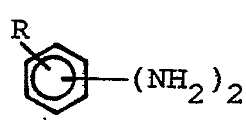

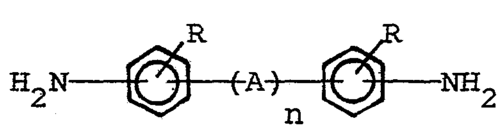

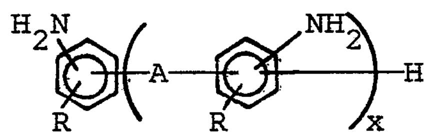

wherein each R is independently hydrogen or a hydrocarbyl or hydrocarbyloxy group containing up to 8 carbon atoms, preferably up to 4 carbon atoms, A is a divalent hydrocarbon group having from 1 to 10 carbon atoms, preferably from 1 to 6 carbon atoms, n has a value of zero or 1 and x has an average value of from 1.1 to 10, preferably from 2 to 4.

Particularly suitable amines include, for example, aniline, o-, m- or p-toluidine, 2,4-xylidine, 3,4-xylidine, 2,5-xylidine, 4-ethylaniline, 3-propylaniline, 1,3-diaminobenzene, 2,4-diaminotoluene, 2,6-diaminotoluene, 4,4'-diamino-diphenyl methane, 2,4,4'-triamino-diphenyl ether, 2,6-diamino naphthalene, 4,4'-bis-methylene diphenylamine, o-, m- or p-anisidine, and mixtures thereof.

Suitable catalysts which can be employed herein include zinc or divalent tin salts of monovalent organic compounds having a pKa value above 2.8, preferably from 4 to 10, and are soluble in the reaction mixture at the reaction conditions.

Suitable such catalysts include, for example, zinc acetate, zinc propionate, zinc octoate, zinc naphthenate, zinc benzoate, zinc pivalate, zinc stearate, zinc itaconate, zinc p-chlorobenzoate, zinc methoxide, zinc phenolate, zinc acetylacetonate, zinc chloride, stannous chloride, stannous octoate, zinc tallate, zinc

neodecanoate, zinc acrylate, zinc formate, zinc chloroacetate, zinc thiodipropionic acid, stannous laurate, and mixtures thereof.

When the organic carbonate is a cyclic organic carbonate such as, for example, ethylene carbonate, or an acyclic carbonate such as, for example, dimethyl carbonate, a zinc or divalent tin salt of trifluoroacetic acid can be employed, even though trifluoroacetic acid has a pKa value below 2.8.

The reaction is generally conducted at a temperature of from 80°C to 300°C, preferably from 120°C to 250°C and most preferably from 130°C to 200°C for a time sufficient to complete the reaction depending upon the particular catalyst, reactants and temperature employed. Usually around 150°C, the time is from one hour to six hours, preferably from two hours to four hours. The reaction generally should be conducted under conditions at which none of the reactants or desired product undergo decomposition.

The quantity of catalyst employed usually depends upon the activity of the particular catalyst. Usually from 0.001 mole to 0.2 mole, preferably from 0.005 mole to 0.05 mole, of catalyst per mole of reactant present in a stoichiometric quantity is suitable.

The reactants can be employed on an equimolar basis or one may be present in an excess of the other up to a 20, preferably from a one to a five moles excess of the other. It is preferred that the organic carbonate reactant be employed in excess of the aromatic amine.

Uses for the desired carbamate reaction product is suitably discussed by Rosenthal et al in U.S. 3,919,279, U.S. 3,919,280 and U.S. 3,962,302.

The following examples are illustrative of the present invention but are not to be construed as to limiting the scope thereof in any manner.

Catalysts employed in the Examples and Comparative Runs are designated as shown below. For salts of monovalent organic compounds, the pKa value listed is the pKa value of the organic compound from which it was derived, e.g., for zinc acetate, the pKa is for acetic acid.

| Catalyst | Designation | pKa |
|---|---|---|
| Zinc chloride | A | |
| Stannous chloride | B | |
| Antimony trichloride | C | |
| Aluminum trichloride | D | |
| Ferric chloride | E | |
| Zinc acetate | F | 4.84 |
| Zinc acetate dihydrate | G | 4.84 |
| Zinc oxyacetate | H | 4.84 |
| Zinc naphthenate | I | 5.00 |
| Zinc octoate | J | 4.85 |
| Stannous octoate | K | 4.85 |
| Zinc propionate | L | 4.88 |
| Zinc salicylate | M | 3.00 |
| Zinc pivalate | N | 5.02 |
| Zinc benzoate | O | 4.17 |
| Zinc acrylate | P | 4.25 |
| Zinc parachlorobenzoate | Q | 4.03 |

| Catalyst | Designation | pKa |
| --- | --- | --- |
| Zinc phenolate | R | 10.00 |
| Zinc formate | S | 3.77 |
| Zinc chloroacetate | T | 2.85 |
| Zinc acetylacetonate | U | 9.00 |
| Zinc oxalate | V | 1.23 |
| Stannous oxalate | W | 1.23 |
| Zinc trifluoroacetate | X | 0.23 |
| Uranium trioxide | Y | |
| Uranium dioxide | Z | |

Zinc oxyacetate (H) is prepared by drying zinc acetate dihydrate in a heated vacuum oven. Zinc naphthenate (I) is a zinc salt of a naphthenic acid containing 8 weight percent zinc. Naphthenic acid is a term used in petroleum chemistry to denote the monocarboxylic acids derived from certain saturated hydrocarbons, specifically 5- and 6-carbon cycloparafins and their alkyl derivatives. Specific acids identified in naphthenic acid are cyclohexyl propionic acid (pKa = 4.91), cyclohexyl butyric acid (pKa = 4.95) and cyclopentane carboxylic acid (pKa = 4.99). Estimated pKa for naphthenic acid is 5. Zinc octoate (J) is a zinc salt of 2-ethylhexanoic acid containing 18 weight percent zinc.

EXAMPLES 1-18 AND COMPARATIVE RUNS A-C

In the following examples and comparative runs, 5 grams (53.7 mmols) of aniline and 25 grams (277.5 mmols) of dimethyl carbonate were mixed with the amount of catalyst shown in Table I and poured into a stainless steel cylindrical container, 1 5/8" (41.3 mm) internal diameter, and 2 1/4" (57.2 mm) internal depth with a wall thickness of 3/16" (4.8 mm). A stainless steel lid screwed over the reactor with a polytetrafluoroethylene O-ring being used as a seal. Mounted on the

lid using stainless steel pipe fittings and parts were a pressure gauge, a pressure relief valve, and a needle valve.

After charging the mixture to the reactor, the lid was sealed by tightening down on the polytetrafluoroethylene O-ring and the reactor was immersed in a hot fluidized sand bath thermostatically controlled at 140°C in the center of the bath. The reactor was heated from between 10 to 15 minutes, then shaken to mix the contents. Then heating was continued for the desired time. After cooling the contents were analyzed for yields of carbamate and by-products.

The quantity of catalysts, reaction conditions and results are given in the following Table I. In Table I, the pressure listed under Reaction Conditions is gauge pressure. Under the headings of Reaction Products, MPC stands for methyl phenyl carbamate, NMA stands for N-methyl aniline, and DPV stands for diphenyl urea. The Mole % Conversion of Aniline to MPC is calculated by dividing the mmols of MPC produced by the mmols of aniline employed (57.3 mmols) and multiplying by 100.

TABLE I

| Example or Comp. Run | Catalyst/ g/mmol | Reaction Conditions Time Hours | Temp °C | Pres. psi (MPa) | Reaction Products MPC g/mmol | NMA g/mmol | DPV g/mmol | Mole % Conversion of Aniline to MPC |
|---|---|---|---|---|---|---|---|---|
| 1 | A/0.34/2.5 | 2 | 140 | 200 (1.38) | 1.86/12.3 | 1.47/13.7 | 0.03/0.1 | 22.91 |
| 2 | B/0.474/2.5 | 2 | 140 | 90 (0.62) | 3.43/22.7 | 0.93/8.7 | 0.066/0.3 | 42.27 |
| A | C/0.57/2.5 | 2 | 140 | 120 (0.83) | 1.25/8.3 | 0.26/2.4 | 0.27/1.3 | 15.46 |
| 3 | F/0.46/2.5 | 2 | 140 | 170 (1.17) | 7.1/47 | 0.024/0.2 | 0.1/0.5 | 87.52 |
| 4 | G/0.55/2.5 | 2 | 140 | 160 (1.10) | 7.0/46.3 | 0.27/2.5 | 0.15/0.7 | 86.22 |
| 5 | H/0.66/2.5 | 2 | 140 | 110 (0.76) | 7.1/47.0 | 0.15/1.4 | 0.12/0.6 | 87.52 |
| 6 | I/2.043/2.5 | 2 | 140 | 110 (0.76) | 7.5/49.6 | 0.1/0.9 | 0.44/2.2 | 92.36 |
| 7 | J/0.185/2.5 | 2 | 200 | 300 (2.07) | 6.66/44 | 0.54/5 | 0.05/0.2 | 82.04 |

TABLE I Con'd

| Example or Comp. Run | Catalyst/ g/mmol | Reaction Conditions: Time Hours | Temp °C | Pres. psi (MPa) | Reaction Products: MPC g/mmol | NMA g/mmol | DPV g/mmol | Mole % Conversion of Aniline to MPC |
|---|---|---|---|---|---|---|---|---|
| 8 | K/1.02/2.5 | 2 | 140 | 140 (0.97) | 2.2/14.5 | 0.39/3.6 | 0.27/1.3 | 27.11 |
| 9 | L/0.53/2.5 | 2 | 140 | 140 (0.97) | 7.59/50.2 | 0.12/1.1 | 0.07/0.3 | 82.68 |
| 10 | M/0.98/2.5 | 2 | 140 | 110 (0.76) | 0.5/3.3 | 0.76/7.1 | 0.20/1.0 | 6.15 |
| 11 | N/1.34/5 | 2 | 140 | 200 (1.38) | 7.05/46.6 | 0.13/1.2 | 0.12/0.6 | 86.78 |
| 12 | O/0.77/2.5 | 2 | 140 | 140 (0.97) | 7.26/48 | 0.28/2.6 | 0.22/1.1 | 89.36 |
| 13 | P/0.519/2.5 | 2 | 140 | 150 (1.03) | 6.72/44.5 | 0.10/0.9 | 0.58/2.8 | 82.87 |
| 14 | Q/0.950/2.5 | 2 | 140 | 160 (1.10) | 6.73/44.5 | 0.55/5.1 | 0.15/0.7 | 82.87 |
| 15 | R/0.63/2.5 | 5 | 140 | 140 (0.97) | 1.8/11.9 | 0.13/1.2 | 0.12/0.6 | 22.16 |

TABLE I Con'd

| Example or Comp. Run | Catalyst/ g/mmol | Reaction Conditions: Time Hours | Reaction Conditions: Temp °C | Reaction Conditions: Pres. psi (MPa) | Reaction Products: MPC g/mmol | Reaction Products: NMA g/mmol | Reaction Products: DPV g/mmol | Mole % Conversion of Aniline to MPC |
|---|---|---|---|---|---|---|---|---|
| 16 | S/0.48/2.5 | 2 | 200 | 450 (3.10) | 2.9/20 | 2.9/2.7 | 0.09/0.4 | 37.24 |
| 17 | T/0.636/2.5 | 2 | 200 | 280 (1.93) | 2.31/15.3 | 0.30/2.8 | 0/0 | 28.46 |
| 18 | X/0.734/2.5 | 2 | 200 | 390 (2.69) | 2.88/19 | 0.84/7.8 | 0.06/0.3 | 35.48 |
| B | Y/0.715/2.5 | 2 | 140 | 150 (1.03) | 0.07/0.5 | 0.2/1.9 | 0/0 | 0.93 |
| C | Z/0.4/1.5 | 2 | 140 | 150 (1.03) | 0.16/1.1 | 0.35/3.3 | 0.13/6 | 2.05 |

0065026

EXAMPLES 21-28 AND COMPARATIVE RUNS D-G

Equimolar amounts of aniline and ethylene carbonate were mixed together. Then 10 gram quantities (5.14 g, 0.0552 mole aniline; 4.86 g, 0.0552 mole ethylene carbonate) of this mixture were weighed into each of a series of 4 dram (15 cc) glass vials. Various catalysts, one per vial, were then weighed into the vials and mixed. The vials were then placed, unsealed, in a metal block of a "Temp Blok" module heater with thermostatic control to hold any desired temperature and the mixture heated for the desired time. Small aliquots could be removed periodically and analyzed for the degree of carbamate formation, if any.

The various catalysts and quantity of catalyst, reaction conditions and results are given in Table II.

TABLE II

| Example or Comp. Run | Catalyst g/mmol | Conditions Time Hours | Temp. °C | Reaction Products HENPC g/mmol | NHEA g/mmol | Mole % Conversion of Aniline to HENPC |
|---|---|---|---|---|---|---|
| 19 | A/0.18/1.3 | 44 | 90 | 3.09/17.1 | 1.10/8.0 | 30.98 |
| 20 | B/0.1/0.53 | 40 | 90 | 3.56/19.6 | 1.11/8.1 | 35.51 |
| D | D/0.06/0.45 | 72 | 90 | 1.58/8.7 | 2.98/21.7 | 15.76 |
| E | E/0.2/1.1 | 40 | 108 | 2.79/15.4 | 1.98/14.4 | 27.9 |
| 21 | F/0.1/0.55 | 40 | 90 | 4.97/27.4 | 1.01/7.4 | 49.64 |
| 22 | K/0.4/0.9 | 72 | 90 | 4.27/23.6 | 0.73/5.3 | 42.75 |
| 23 | M/0.2/2.5 | 36 | 108 | 2.26/12.5 | 1.0/7.3 | 22.64 |
| 24 | P/0.2/1.5 | 36 | 108 | 2.33/12.9 | 0.88/6.4 | 23.37 |
| 25 | S/0.2/1 | 36 | 108 | 4.46/24.6 | 1.77/12.9 | 44.57 |
| 26 | T/0.2/0.8 | 36 | 108 | 2.17/12.0 | 3.48/25.4 | 21.74 |
| 27 | U/0.05/0.3 | 44 | 90 | 3.54/19.5 | 1.02/7.4 | 35.33 |
| F | V/0.2/1 | 36 | 108 | 0.19/1 | 3.47/25.3 | 1.81 |
| G | W/0.1/0.48 | 40 | 90 | 0.18/1 | 1.21/8.8 | 1.81 |
| 28 | X/0.2/0.7 | 36 | 108 | 4.13/22.8 | 1.11/8.1 | 41.3 |

EXAMPLES 29-36

These examples were prepared in the same manner as those for Examples 1-18 except that different amines and a different carbonate were employed. The reactants, reaction conditions and results are given in the following Table III. The amines and their designations and the carbonates and their designations are listed below.

| Amines | Designation |
|---|---|
| 2,6-Diaminotoluene | AA |
| 2,4-Diaminotoluene | BB |
| p-Toluidene | CC |
| o-Anisidine | DD |
| m-Anisidene | EE |
| p-Anisidene | FF |
| Aniline | GG |

| Carbonates | |
|---|---|
| Dimethyl carbonate | HH |
| Diethyl carbonate | II |

0065026

TABLE III

| Example | Catalyst/ g/mmol | Amine/ g/mmol | Carbonate/ g/mmol | Reaction Conditions Time Hours | Temp. °C | Pres. psi (MPa) | Carbamate g/mmol | Mole % Conversion of Amine to Carbamate |
|---|---|---|---|---|---|---|---|---|
| 29 | F/0.46/2.5 | AA/3.4/27.9 | HH/25/277.5 | 4 | 140 | 240 (1.65) | 2.37/10 | 36.0 |
| 30 | F/0.46/2.5 | DD/6.2/60 | HH/22.5/250 | 2 | 160 | 220 (1.52) | 5.6/31 | 62 |
| 31 | F/0.46/2.5 | EE/6.2/50 | HH/22.5/250 | 2 | 160 | 220 (1.52) | 6.6/37 | 74 |
| 32 | F/0.46/2.5 | FF/6.2/50 | HH/22.5/250 | 2 | 160 | 220 (1.52) | 8.0/44 | 88 |
| 33 | J/1.82/5 | AA/3.45/28.3 | HH/25/277.5 | 4 | 160 | 220 (1.52) | 2.54/10.7 | 37.8 |
| 34 | J/1.82/5 | BB/3.45/28.3 | HH/25/277.5 | 4 | 160 | 220 (1.52) | 3.92/16.5 | 58.3 |
| 35 | J/0.911/2.5 | GG/5/53.7 | II/25/212 | 2 | 200 | 180 (1.24) | 4.8/27 | 54 |
| 36 | L/0.534/2.5 | CC/5.75/53.7 | II/32.8/277.5 | 4 | 140 | 100 (0.76) | 6.42/38.7 | 72 |

Zinc acetate (F) was also found to be suitable for catalyzing the reaction between dimethyl carbonate and methylene dianiline, o-phenylenediamine, m-phenylenediamine, p-phenylenediamine, and an 80/20 mixutre of 2,4-/2,6-diamino toluene.

Zinc naphthenate (I) was also found to be suitable for catalyzing the reaction between dimethyl carbonate and 2,6-diamino toluene and an 80/20 mixture of 2,4-/2,6-diamino toluene.

Zinc octoate (J) was found to not catalyze the reaction between dimethyl carbonate and 2,4,6--tribromoaniline.

1. A process for preparing a carbamate from an organic carbonate and an aromatic amine in the presence of catalytic quantity of a Lewis acid catalyst characterized by employing as the Lewis acid catalyst a catalyst which is soluble in the reaction mixture at the reaction conditions employed and which is at least one member selected from the group consisting of a zinc or divalent tin halide, a zinc or divalent tin salt of a monovalent organic compound which has a pKa value of at least 2.8, and a zinc or divalent tin salt of trifluoroacetic acid.

2. The process of Claim 1 characterized in that the compound has a pKa of from 4 to 10.

3. The process of Claim 1 characterized in that the catalyst is selected from zinc naphthenate, zinc acetate, zinc propionate, zinc octoate or mixtures thereof.

European Patent Office

# EUROPEAN SEARCH REPORT

0065026

Application number

EP 81103810.8

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| D,X | US - A - 3 763 217 (BRILL)<br>* Column 3, line 22; claims 1-3 *<br>---- | 1 | C 07 C 125/06<br>B 01 J 27/08<br>B 01 J 31/02<br>B 01 J 31/04<br>B 01 J 31/26 |
| | | | TECHNICAL FIELDS SEARCHED (Int. Cl.3) |
| | | | C 07 C 125/00<br>B 01 J |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 11-12-1981 | HERING |